# EUROPÄISCHE PATENTSCHRIFT

(11) **EP 1 802 342 B1**
(45) Veröffentlichungstag und Bekanntmachung des Hinweises auf die Patenterteilung: **31.07.2013**
(21) Anmeldenummer: 05804396.9
(22) Anmeldetag: 07.10.2005
(51) Int. Cl.: A61K 39/395, A61K 39/44, C07K 14/47

(54) **IMMUNADSORBER FÜR DIE BEHANDLUNG VON ENTZÜNDUNGEN**
IMMUNOADSORBER FOR TREATING INFLAMMATIONS
IMMUNOADSORBANTS POUR LE TRAITEMENT D'INFLAMMATIONS

(30) Priorität: 20.10.2004 DE 102004051216; 20.01.2005 DE 102005003190
(43) Veröffentlichungstag der Anmeldung: 04.07.2007
(73) Patentinhaber: Heinrich, Hans-Werner, Prof. Dr., 17489 Greifswald (DE)
(72) Erfinder: Heinrich, Hans-Werner, Prof. Dr., 17489 Greifswald (DE)
(74) Vertreter: Baumbach, Friedrich
(86) Internationale Anmeldenummer: PCT/DE2005/001797
(87) Internationale Veröffentlichungsnummer: WO 2006/042507

(56) Entgegenhaltungen:
- EP-A- 0 082 345
- EP-A- 0 230 247
- EP-A- 0 300 740
- WO-A-00/58005
- DE-A1- 19 818 790
- DATABASE WPI Week 198917, Derwent Publications Ltd., London, GB; AN 1989-123616 & EP 0 312 645 A (PROGEN BIOTECHNIK) 26 April 1989

## Beschreibung

Die Erfindung betrifft immunologische Adsorber für die Behandlung von Entzündungserkrankungen, insbesondere für die Entfernung von Komplementfaktoren sowie gegebenenfalls von Interleukinen und TNF aus Körperflüssigkeiten, Verfahren zu ihrer Herstellung und ihre Verwendung.

Ein Immunoadsorber zur Sepsistherapie ist bereits aus WO 00/58005 bekannt. Er ist gekennzeichnet durch Trägermaterialien aus organischen oder synthetischen Polymeren, an die sowohl poly- oder monoklonale Antikörper gebunden sind, die gegen die Komplementfaktoren C3a und/oder C5a gerichtet sind, als auch Antikörper, die gegen Lipopolysaccharide (LPS) gerichtet sind. In einer bevorzugten Ausführung sind auch Antikörper, die gegen weitere Sepsis-Mediatoren gerichtet sind, an die Träger gebunden.
Er dient zur Entfernung von Komplementfaktoren und Lipopolysacchariden (LPS) sowie gegebenenfalls zur Entfernung von weiteren Sepsis-Mediatoren, wie von TNF und Interleukinen aus Körperflüssigkeiten. Damit kann er beispielsweise für die Plasmapherese bei Patienten mit Sepsis eingesetzt werden.

Der Nachteil dieses Adsorbersystems besteht darin, das seine Anwendung auf die durch gramnegative Bakterien hervorgerufene Ganzkörperentzündung "Sepsis" beschränkt ist. Die Herstellung polyspezifische Antikörper gegen die unterschiedlichen Lipopolysaccharide (LPS) ist zudem aufwendig und teuer. Die Behandlung von Patienten mit diesem Adsorber, die an einer Sepsis leiden, die durch gramnegative Bakterien (z.B. Escherichia coli) hervorgerufen wird, bringt gute Ergebnisse. Für die Therapie der Sepsis, die durch andere Ursachen hervorgerufen wird, ist dieser LPS-Adsorber zumindest nicht notwendig, auf jedem Falle aber zu teuer. Der oben genannte Adsorber hat naturgemäß sein Einsatzgebiet in der Sepsis-Behandlung. Für andere Erkrankungen, die mit Entzündungen einhergehen, ist er nicht geeignet.

Der Erfindung liegt die Aufgabe zu Grunde, ein modular aufgebautes Immunadsorptionssystem insbesondere für die extrakorporale Detoxifikation zu entwickeln, das es ermöglicht, patientenspezifisch Plasma- und Gewebespiegel zu reduzieren, welches die oben genannten Mängel nicht aufweist.

Diese Aufgabe wird gemäß dem Hauptanspruch gelöst, die Unteransprüche sind Vorzugsvarianten.

Eine besonders vorteilhafte Ausführungsform der Erfindung stellt ein Immunadsorber mit Antikörpern gegen C5a und IL6 dar. Die C5a-Entfernung ist wichtig, da dies der wirkungsvollste Entzündungs-Faktor aus der Komplement-Kaskade ist und IL6, weil dieses Interleukin eine zentrale Funktion in der pro-Entzündungs-Regulation einnimmt.

Bei den eingesetzten Antikörpern handelt es sich bevorzugt um polyklonale Antikörper, besonders bevorzugt um aviäre Antikörper des Typs IgY. Die Antikörper gegen Sepsis-Mediatoren sind entsprechend des Zustandes der Dysregulation enthalten.

Gemäß der Erfindung handelt es sich dabei um Antikörper, die gegen TNF, IL1, IL6, IL8 und/oder IL10 gerichtet sind.

Bevorzugte Antikörper gegen den Komplementfaktor C3a weisen spezifische Aktivität gegen mindestens eine der folgenden Peptidsequenzen auf:

NH₂-KCCEDGMRQNPMR-COOH

NH₂-RFSCQRRTRFISL-COOH

NH₂-ITELRRQHARAS-COOH

Gemäß Anspruch 1 besteht der immunlogische Adsorber aus organischen und/oder synthetischen polymeren Trägermatrialien, an die sowohl poly- und/oder monoklonale Antikörper gebunden sind, die gegen die Komplementfaktoren
- C3a und C5a sowie ggf. mit Antikörpern, die gegen TNF, IL1, IL6, 1L8 und/oder IL10, oder
- C3a sowie ggf. mit Antikörpern, die gegen TNF, IL1, IL6, IL8 und/oder IL10, oder
- C5a sowie mit Antikörpern, die gegen TNF, IL1, IL6, IL8 und/oder IL10 gerichtet sind.

Bevorzugte Antikörper gegen den Komplementfaktor C5a besitzen spezifische Aktivität gegen mindestens eine der folgenden Peptidsequenzen:

NH₂-QADYKDDDDKLPAE-COOH

NH₂-DDKLPAEGLDIENS-COOH

Bevorzugte Antikörper gegen IL1α/β besitzen spezifische Aktivität gegen mindestens eine der folgende Peptidsequenzen

NH₂-NCYSENEEDSSSID-COOH

NH₂-GAYKSSKDDAKIT-COOH

NH₂-WETHGTKNYFTS-COOH

NH₂-RISDHHYSKGFRQA-COOH

NH₂-VQGEESNDKIPVA-COOH

NH₂-ESVDPKNYPKKKMEKRF-COOH

Bevorzugte Antikörper gegen IL6 besitzen spezifische Aktivität gegen mindestens eine der folgende Peptidsequenzen

NH₂-APHRQPLTSSERIDKQI-COOH

NH₂-QNRFESSEEQARA-COOH

NH₂-AITTPDPTTNAS-COOH

Bevorzugte Antikörper gegen IL10 besitzen spezifische Aktivität gegen mindestens eine der folgende Peptidsequenzen

NH₂-SPGQGTQSENSCT-COOH

NH₂-QMKDQLDNLLLKES-COOH

NH₂-MPQAENQDPDIKA-COOH

NH₂-LPCENKSKAVEQ-COOH

Bevorzugte Antikörper gegen TNFα besitzen spezifische Aktivität gegen mindestens eine der folgende Peptidsequenzen

NH₂-VRSSSRTPSDKPVA-COOH

NH₂-KSPCQRETPEGAEAKPW-COOH

Der erfindungsgemäße Immunadsorber weist als Trägermaterialien an sich übliche Membranen oder Partikel aus organischen oder synthetischen Polymeren auf, so z.B. aus Polystyrolen, Kohlenhydraten, wie z.B. Zellulose- oder Agarosederivate, oder aus Acrylaten, wobei die spezifischen Antikörper kovalent an diese gebunden oder über Spacer oder Linker an sie fixiert sind.

Die Herstellung der erfindungsgemäßen Immunadsorber erfolgt durch an sich bekannte Methoden, indem die Antikörper, die gegen
- C3a und C5a sowie ggf. gegen TNF, IL1, IL6, IL8 und/oder IL10, oder
- C3a sowie ggf. gegen TNF, IL1, IL6, IL8 und/oder IL10, oder
- C5a sowie gegen TNF, IL1, IL6, IL8 und/oder IL10
gerichtet sind, kovalent oder adsorptiv an die Trägermaterialen aus organischen oder synthetischen Polymeren gekoppelt werden.

Die spezifischen Antikörper werden durch an sich bekannte Immunisierung vorzugsweise von Kleinsäugern, wie Mäusen, Ratten oder Kaninchen oder Vögeln, wie z.B. Hühnern, mit den entsprechenden Antigenen hergestellt.

Gegenstand der Erfindung ist auch die Verwendung der Immunadsorber in Vorrichtungen zur Entfernung von Komplementfaktoren und ggf. von weiteren Mediatoren aus Körperflüssigkeiten, wie Blutplasma, in Abhängigkeit der patientenspezifischen Situation.

Bevorzugt werden die Immunadsorber in der Sepsistherapie für die Plasmapherese bei Patienten mit Sepsis oder septischem Schock eingesetzt.

Obwohl für die meisten Substanzen Antikörper verfügbar sind, die nach bekannten Methoden an die unterschiedlichen Träger gekoppelt werden können, werden bevorzugt aviäre Antikörper verwendet, da diese im Gegensatz zu Säuger-Antikörpern das Komplementsystem nicht aktivieren. Da die aktivierenden Eigenschaften an den F_{c}-Teil der Säuger-Antikörper gebunden sind, kann prinzipiell auch das mit Papain abgespaltene F_{ab}-Fragment verwendet werden.

Nach derzeitigem Kenntnisstand haben immobilisierte aviäre Antikörper keinerlei unspezifische Wirkungen auf das Abwehrsystem des Menschen. Vögel, vorzugsweise Hühner werden mit üblichen Verfahren ohne oder mit Verwendung von Adjuvantien immunisiert. Die spezifischen Immunglobuline werden im Eidotter ausgeschieden und können hieraus mit üblichen Methoden isoliert werden. Sie werden mit bekannten Verfahren kovalent über den Fc-Teil an Mikropartikel oder Membranen gebunden.

Mit dem erfindungsgemäßen Immunadsorptionssystem für die extrakorporale Detoxifikation steht erstmals ein selektives System zur Verfügung, welches patientenspezifisch einsetzbar ist und durch das Fehlregulationen des Immunsystems behoben werden können.

Die Erfindung soll nachfolgend durch Beispiele näher erläutert werden.

### Ausführungsbeispiele:

### Beispiel 1:

### a) Aktivierung eines Trägers:

Die nach Beispiel 3 gereinigten IgY werden kovalent an einen geeigneten Träger gebunden. Dazu kann z.B. Sepharose wie nachstehend beschrieben, aktiviert werden (H.-F.Boeden, W.Büttner, C.Rupprich, D.Büttner, S.Heinrich, M.Becker, M.Holtzhauer (1992) Makromol. Chem. 193, 865-887):

Der Agarose-Träger wird stufenweise, d.h. durch eine um 20 % schrittweise sich erhöhende Menge an Aceton überführt. Zum Schluß wird der Träger in einem fünffachen Bettvolumen mit wasserfreiem Aceton in einem verschlossenen Gefäß über Nacht stehen gelassen und nochmals mit 5 bis 10 Vol. wasserfreiem Aceton gewaschen und kurz auf einer G2-Fritte abgesaugt. Zu 10 ml sedimentiertem Träger werden 400 mg N-(Chlorcarbonyloxy)-5-norbornen-2,3-dicarboximid (CICOONB) in 10 ml wasserfreiem Aceton p.a. gegeben. Unter Schütteln wird innerhalb von 15 Minuten eine Lösung von 280 µl Triethylamin und 20 mg 4-Dimethylamino-pyridin (DMAP) in 5 ml trockenem Aceton tropfenweise zugegeben (Molverhältnis CICOONB:Triethylamin:DMAP 1:1,2:0,1). Man schüttelt anschließend weitere 15 Minuten und wäscht dann den Träger mit ca. 200 ml wasserfreiem Aceton.

### b) Kopplung der Immunglobuline an einen festen Träger

Die nach Beispiel 1a) aktivierte Polysaccharid-Matrix (Gel) wird stufenweise in ein wäßriges Medium überführt und dann sofort in die Kopplungslösung, die den Liganden enthält, eingerührt. Als Kopplungspuffer wird Citratpuffer pH 4,2 verwendet. Die Kopplung erfolgt unter leichtem Schütteln für 2 h bei Raumtemperatur. Freie Bindungen werden anschließend durch Zugabe von Ethanolamin blockiert. In der Tab. 1 sind die konkreten Bedingungen für die einzelnen Antikörper dargestellt.

**Tabelle 1**

| **Gel Nr.** | **Chicken-Ak (IgY)** | **mg** | **mg/ml** | **Ak-Lösung (ml)** | **ml Kopplungspuffer (Citrat, 0,1 M, pH 4,2)** | **Ethanolamin 1 M (ml)** | **feuchtes Gel (g)** |
|---|---|---|---|---|---|---|---|
| 1 | Cha. C3a | 8,3 | 9,6,7 | 0,6 | 4,4 | 0,5 | 5,77 |
| 2 | Cha. C5a | 10,3 | 10,2 | 1 | 4 | 0,5 | 5,63 |
| 3 | Cha. IL6 | 9,5 | 9,8 | 1 | 4 | 0,5 | 5,52 |
| 8 | Kontrolle | 0 | 0 | 0 | 5 | 0,5 | 5,60 |

### Beispiel 2

Die nach Beispiel 1 immobilisierten Antikörper werden benutzt, um aus flüssigen Medien wie Pufferlösungen, Serum oder Blutplasma Interleukin 6, oder Komplementfaktoren zu entfernen.
Dazu werden die Träger gewaschen, in einen physiologischen Puffer (PBS) überführt und luftblasenfrei in Plastik- oder Glassäulen gepackt. Die Anordnung wird durch Anschluß an eine Chromatografieeinrichtung komplettiert. Das zu adsorbierende Probenmaterial (mit den Antigenen dotierter Puffer, Serum- oder Blutplasmaproben, dotiert oder mit natürlichem Antigengehalt) kann nun durch Schwerkraft oder mit einer geeigneten Pumpe über die immobilisierten, für die aufgeführten Antigene spezifischen Antikörper geleitet werden. Die vorhandenen Antigene werden von den IgY erkannt, fest gebunden und somit aus dem die Säule durchströmenden Medium entfernt. Der Nachweis der Wirksamkeit erfolgt durch Analyse (ELISA) des Säulendurchlaufs, dessen Antigengehalt vermindert ist. Nach Waschen der Säule mit physiologischem Puffer erfolgt die Desorption des gebundenen Antigens mit geeigneten Elutionsmitteln (0,1 M Citratpuffer pH 3), Fraktionierung und Analyse des Eluates. Der quantitative Nachweis der Antigene wird zur Kapazitätsbestimmung des Immunadsorbents genutzt.

| **Antigen** | **Experiment** | **Kopplungsdichte** | **Gelvolumen** | **Volumen/ Medium** | **adsorbierte Menge** |
|---|---|---|---|---|---|
| | | **[mg/ml Gel]** | **[ml]** | **[ml]** | |
| C3a | batch | 10 | 0,5 | 2 / Plasma | 12 µg |
| | zyklisch | 10 | 0,1 | 5 / Plasma | 2,6 µg nach 3 Zyklen |
| C5a | batch | 10 | 0,5 | 2 / Plasma | 4 µg |
| | zyklisch | 10 | 0,5 | 25 / Plasma | 2 µg nach 10 Zyklen |
| | | | | | 1,7 µg nach 3 Zyklen |
| IL6 | batch | 5 | 0,1 | 1/ Plasma | 133 ng |
| | Affinitätschromatografie | 2 | 0,1 | 1/ PBS+1%BSA | 330 ng |
| | (1 Zyklus) | | | | |
| | zyklisch | 10 | 1 | 50/ PBS+1%BSA | 100 ng |
| | | | | | 94 ng nach 3 Zyklen |
| | | 5 | 1 | 50/ PBS+1%BSA | 93 ng |
| | | | | | 74 ng nach 3 Zyklen |

### Beispiel 3

Die nach Beispiel 1 immobilisierten Antikörper werden benutzt, um aus Patientenblutplasma Interleukin 6 und aktiviertes Komplement 5a zu entfernen.
Dazu werden die Träger gewaschen, in einen physiologischen Puffer (PBS) überführt und luftblasenfrei in Plastikgehäuse gepackt. Die Adsorbermodule enthalten 60 ml Gel. Zwei verschiedenen Adsorber enthalten Antikörper, die jeweils gegen C5a (10 mg IgY/ml Sepharose-Gel (entspricht einer Adsorptionskapazität von 400 µg/Säule) bzw. IL6 (5 mg IgY/ml Sepharose-Gel (entspricht einer Adsorptionskapazität von 240 µg/Säule)) gerichtet sind. Die Module sind in Reihe geschaltet.

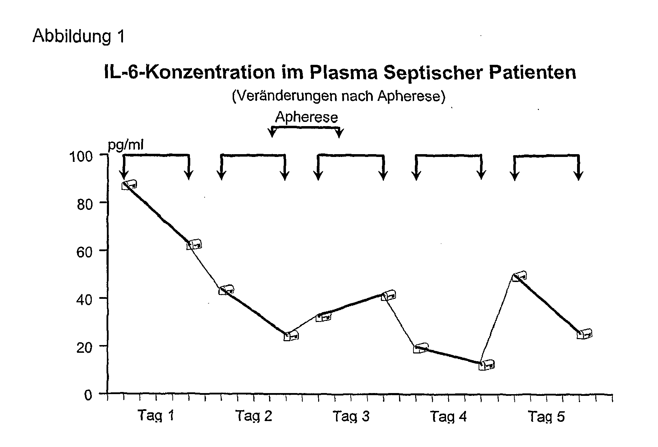

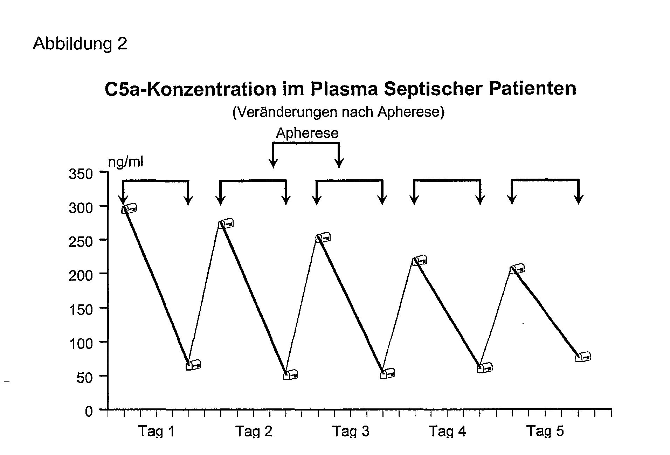

Folgende Abreicherungskinetiken konnten an 5 aufeinanderfolgenden Tagen (5 Liter Plasmabehandlung pro Tag) ermittelt werden (Abbildungen 1 und 2):

## Patentansprüche

1. Immunologischer Adsorber zur Anwendung in einem Verfahren für die Behandlung von Entzündungen bestehend aus
Trägermaterialien aus organischen oder synthetischen Polymeren mit gebundenen poly- oder monoklonalen Antikörpern, die gegen die
Komplementfaktoren
- C3a und C5a sowie ggf. mit Antikörpern, die gegen TNF, IL1, IL6, IL8 und/oder IL10, oder
- C3a sowie ggf. mit Antikörpern, die gegen TNF, IL1, IL6, IL8 und/oder IL10, oder
- C5a sowie mit Antikörpern, die gegen TNF, IL1, IL6, IL8 und/oder IL10 gerichtet sind.

2. Immunologische Adsorber nach Anspruch 1 zur Anwendung gemäß Anspruch 1, **dadurch gekennzeichnet, dass** die Antikörper polyklonale Antikörper sind.

3. Immunologische Adsorber nach Anspruch 2 zur Anwendung gemäß Anspruch 1, **dadurch gekennzeichnet, dass** die Antikörper aviäre Antikörper des Typs IgY sind.

4. Immunologische Adsorber nach Anspruch 1 bis 3 zur Anwendung gemäß Anspruch 1, **dadurch gekennzeichnet, dass** weitere Antikörper gegen Entzündungs-Mediatoren entsprechend des Regulationszustandes der Entzündungsreaktion enthalten sind, wobei diese Antikörper gegen TNF, IL1, IL6, IL8 und/oder IL10 gerichtet sind.

5. Immunologischer Adsorber nach Anspruch 1 und 4 zur Anwendung gemäß Anspruch 1, **dadurch gekennzeichnet, dass** C3a-spezifische Antikörper und C5a-spezifische Antikörper enthalten sind.

6. Immunologischer Adsorber nach Anspruch 1 und 4 zur Anwendung gemäß Anspruch 1, **dadurch gekennzeichnet, dass** Antikörper, die gegen C3a und/oder C5a gerichtet sind, und TNF-spezifische Antikörper enthalten sind.

7. Immunologischer Adsorber nach Anspruch 1 und 4 zur Anwendung gemäß Anspruch 1, **dadurch gekennzeichnet, dass** Antikörper, die gegen C3a und/oder C5a gerichtet sind, und IL1-spezifische Antikörper enthalten sind.

8. Immunologischer Adsorber nach Anspruch 1 und 4 zur Anwendung gemäß Anspruch 1, **dadurch gekennzeichnet, dass** Antikörper, die gegen C3a und/oder C5a gerichtet sind, und IL6-spezifische Antikörper enthalten sind.

9. Immunologischer Adsorber nach Anspruch 1 und 4 zur Anwendung gemäß Anspruch 1, **dadurch gekennzeichnet, dass** Antikörper, die gegen C3a und/oder C5a gerichtet sind, und IL8-spezifische Antikörper enthalten sind.

10. Immunologischer Adsorber nach Anspruch 1 und 4 zur Anwendung gemäß Anspruch 1, **dadurch gekennzeichnet, dass** Antikörper, die gegen C3a und/oder C5a gerichtet sind, und IL10-spezifische Antikörper enthalten sind.

11. Immunologischer Adsorber nach Anspruch 1-10 zur Anwendung gemäß Anspruch 1, **dadurch gekennzeichnet, dass** die gebundenen Antikörper gegen mindestens eine der folgenden Peptidsequenzen der Komplementfaktoren C3a und C5a
C3a:
NH₂-KCCEDGMRQNPMR-COOH
NH₂-RFSCQRRTRFISL-COOH
NH₂-ITELRRQHARAS-COOH
C5a:
NH₂-QADYKDDDDKLPAE-COOH
NH₂-DDKLPAEGLDIENS-COOH
gerichtet sind.

12. Immunologischer Adsorber nach Anspruch 1-4 und 7 zur Anwendung gemäß Anspruch 1, **dadurch gekennzeichnet**, das die gebundenen Antikörper gegen mindestens eine der folgenden Peptidsequenzen der Interleukine 1α und 1β
IL1α:
NH2-NCYSENEEDSSSID-COOH
NH₂-GAYKSSKDDAKIT-COOH
NH₂-WETHGTKNYFTS-COOH
IL1β:
NH₂-RISDHHYSKGFRQA-COOH
NH₂-VQGEESNDKIPVA-COOH
NH₂-ESVDPKNYPKKKMEKRF-COOH
gerichtet sind.

13. Immunologischer Adsorber nach Anspruch 1-4 und 8 zur Anwendung gemäß Anspruch 1, **dadurch gekennzeichnet, dass** die gebundenen Antikörper gegen mindestens eine der folgenden Peptidsequenzen des Interleukin 6
IL6:
NH₂-APHRQPLTSSERIDKQI-COOH
NH₂-QNRFESSEEQARA-COOH
NH₂-AITTPDPTTNAS-COOH
gerichtet sind.

14. Immunologischer Adsorber nach Anspruch 1-4 und 10 zur Anwendung gemäß Anspruch 1, **dadurch gekennzeichnet, dass** die gebundenen Antikörper gegen mindestens eine der folgenden Peptidsequenzen des Interleukin 10
IL10:
NH₂-SPGQGTQSENSCT-COOH
NH₂-QMKDQLDNLLLKES-CCOH
NH₂-MPQAENQDPDIKA-COOH
NH₂-LPCENKSKAVEQ-COOH
gerichtet sind.

15. Immunologischer Adsorber nach Anspruch 1-4 und 6 zur Anwendung gemäß Anspruch 1, **dadurch gekennzeichnet, dass** die gebundenen Antikörper gegen mindestens eine der folgenden Peptidsequenzen
TNFα:
NH₂-VRSSSRTPSDKPVA-COOH
NH₂-KSPCQRETPEGAEAKPW-COOH
gerichtet sind.

16. Immunologischer Adsorber nach den Ansprüchen 1 bis 15 zur Anwendung gemäß Anspruch 1, **dadurch gekennzeichnet, dass** das organische oder synthetische Trägermaterial aus Membranen oder Partikeln aus Polystyrolen, Kohlenhydraten, wie Zellulose- oder Agarosederivaten, oder Acrylaten besteht.

17. Immunologischer Adsorber nach den Ansprüchen 1 bis 16 zur Anwendung gemäß Anspruch 1, **dadurch gekennzeichnet, dass** die spezifischen Antikörper kovalent an die Membranen oder Partikel gebunden sind.

18. Immunologischer Adsorber nach den Ansprüchen 1 bis 16 zur Anwendung gemäß Anspruch 1, **dadurch gekennzeichnet, dass** die Antikörper über Spacer oder Linker an die Trägermaterialien fixiert sind.

19. Verfahren zur Herstellung von Immunadsorbern gemäß Anspruch 1 bis 18, **dadurch gekennzeichnet, dass** an Trägermaterialen aus organischen oder synthetischen Polymeren Antikörper, die gegen
- C3a und C5a sowie ggf. gegen TNF, IL1, IL6, IL8 und/oder IL10, oder
- C5a sowie gegen TNF, IL1, IL6, IL8 und/oder IL10
gerichtet sind, kovalent oder adsorptiv gekoppelt werden.

20. Verfahren nach Anspruch 19, **dadurch gekennzeichnet, dass** die Antikörper durch Immunisierung vorzugsweise von Kleinsäugern, wie Mäusen, Ratten oder Kaninchen, oder Vögeln, wie Hühnern, mit den entsprechenden Antigenen hergestellt werden.

21. Verwendung von immunologischen Adsorbern gemäß Anspruch 1 bis 18 als wirksamer Bestandteil einer Vorrichtung zur Entfernung von Komplementfaktoren und ggf. weiteren Mediatoren in patientenspezifischer Kombination aus Körperflüssigkeiten.

22. Verwendung nach Anspruch 21, **dadurch gekennzeichnet, dass** die immunologischen Adsorber als Bestandteil einer Vorrichtung zur Behandlung von Patienten mit Sepsis oder septischen Schock sowie anderen Krankheiten, die mit Entzündungen einhergehen verwendet werden.

## Claims

1. Immunological adsorber for use in a method for the treatment of inflammations, consisting of carrier materials of organic or synthetic polymers with bound polyclonal or monoclonal antibodies directed against the complementary factors
- C3a and C5a and also, if applicable, with antibodies directed against TNF, IL1, IL6, IL8 and/or IL10, or
- C3a and also, if applicable, with antibodies directed against TNF, IL1, IL6, IL8 and/or IL10, or
- C5a and also with antibodies directed against TNF, IL1, IL6, IL8 and/or IL10.

2. Immunological adsorbers according to Claim 1, **characterized in that** the antibodies are polyclonal antibodies.

3. Immunological adsorbers according to Claim 2, **characterized in that** the antibodies are aviary antibodies of type IgY.

4. Immunological adsorbers according to any one of Claims 1 - 3 for use according to claim 1, **characterized in that** further antibodies against inflammation mediators are contained in accordance with the regulation condition of the inflammation reaction, wherein these antibodies are directed against TNF, IL1, IL6, IL8 and/or IL10.

5. Immunological adsorbers according to Claims 1 and 4 for use according to claim 1, **characterized in that** C3a-specific antibodies and C5a-specific antibodies are contained.

6. Immunological adsorbers according to Claims 1 and 4 for use according to claim 1, **characterized in that** antibodies directed against C3a and/or C5a and TNF-specific antibodies are contained.

7. Immunological adsorbers according to Claims 1 and 4 for use according to claim 1, **characterized in that** antibodies directed against C3a and/or C5a and IL1-specific antibodies are contained.

8. Immunological adsorbers according to Claims 1 and 4 for use according to claim 1, **characterized in that** antibodies directed against C3a and/or C5a and IL6-specific antibodies are contained.

9. Immunological adsorbers according to Claims 1 and 4 for use according to claim 1, **characterized in that** antibodies directed against C3a and/or C5a and IL8-specific antibodies are contained.

10. Immunological adsorbers according to Claims 1 and 4 for use according to claim 1, **characterized in that** antibodies directed against C3a and/or C5a and IL10-specific antibodies are contained.

11. Immunological adsorbers according to any one of Claims 1 - 10 for use according to claim 1, **characterized in that** the bound antibodies are directed against at least one of the following peptide sequences of the complementary factors C3a and C5a:
C3a:
NH₂-KCCEDGMRQNPMR-COOH
NH₂-RFSCQRRTRFISL-COOH
NH₂-ITELRRQHARAS-COOH
C5a:
NH₂-QADYKDDDDKLPAE-COOH
NH₂-DDKLPAEGLDIENS-COOH

12. Immunological adsorbers according to any one of Claims 1 - 4 and 7 for use according to claim 1, **characterized in that** the bound antibodies are directed against at least one of the following peptide sequences of interleukins 1α and 1β:
IL1α:
NH₂-NCYSENEEDSSSID-COOH
NH₂-GAYKSSKDDAKIT-COOH
NH₂-WETHGTKNYFTS-COOH
IL1β:
NH₂-RISDHHYSKGFRQA-COOH
NH₂-VQGEESNDKIPVA-COOH
NH₂-ESVDPKNYPKKKMEKRF-COOH

13. Immunological adsorbers according to any one of Claims 1 - 4 and 8 for use according to claim 1, **characterized in that** the bound antibodies are directed against at least one of the following peptide sequences of interleukin 6:
IL6:
NH₂-APHRQPLTSSERIDKQI-COOH
NH₂-QNRFESSEEQARA-COOH
NH₂-AITTPDPTTNAS-COOH

14. Immunological adsorbers according to any one of Claims 1 - 4 and 10 for use according to claim 1, **characterized in that** the bound antibodies are directed against at least one of the following peptide sequences of interleukin 10:
IL10:
NH₂-SPGQGTQSENSCT-COOH
NH₂-QMKDQLDNLLLKES-COOH
NH₂-MPQAENQDPDIKA-COOH
NH₂-LPCENKSKAVEQ-COOH

15. Immunological adsorbers according to any one of Claims 1 - 4 and 6 for use according to claim 1, **characterized in that** the bound antibodies are directed against at least one of the following peptide sequences:
TNFα: NH2-VRSSSRTPSDKPVA-COOH
NH2-KSPCQRETPEGAEAKPW-COOH

16. Immunological adsorbers according to any one of Claims 1 - 15 for use according to claim 1, **characterized in that** the organic or synthetic carrier material comprises membranes or particles of polystyrols, carbohydrates, such as cellulose or agarose derivatives, or acrylates.

17. Immunological adsorbers according to any one of Claims 1 - 16 for use according to claim 1, **characterized in that** the specific antibodies are covalently bound to the membranes or particles.

18. Immunological adsorbers according to any one of Claims 1 - 16 for use according to claim 1, **characterized in that** the antibodies are fixed to the carrier materials via spacers or linkers.

19. Method for the production of immuno-adsorbers according to any one of Claims 1 to 18, **characterized in that** carrier materials of organic or synthetic polymers were covalently or adsorptively coupled to antibodies directed against
- C3a and C5a and also, if applicable, against TNF, IL1, IL6, IL8 and/or IL10, or
- C5a and also against TNF, IL1, IL6, IL8 and/or IL10

20. Method according to Claim 19, **characterized in that** the antibodies are produced by immunization preferably of small mammals, such as mice, rats or rabbits, or of birds, such as chickens, with the corresponding antigens.

21. Use of immunological adsorbers according to any one of Claims 1-18 as an effective integral part of a device for the removal of complementary factors and, if applicable, further mediators from body fluids in a patient-specific combination.

22. Use according to Claim 21, **characterized in that** the immunological adsorbers as a part of a device are used for the treatment of patients with sepsis or septic shock and also other diseases connected with inflammations.

## Revendications

1. Absorbant immunitaire pour une utilisation dans un procédé destiné au traitement d'inflammations, composé de
matériaux supports en polymères organiques ou synthétiques avec des anticorps polyclonaux ou monoclonaux liés qui sont orientés contre les facteurs complémentaires
- C3a et C5a ainsi que, le cas échéant, avec des anticorps qui sont orientés contre TNF, IL1, IL6, IL8 et/ou IL10, ou
- C3a et, le cas échéant, avec des anticorps qui sont orientés contre TNF, IL1, IL6, IL8 et/ou IL10, ou
- C5a et avec des anticorps qui sont orientés contre TNF, IL1, IL6, IL8 et/ou IL10.

2. Absorbants immunitaires selon la revendication n°1 pour une utilisation conformément à la revendication n°1, **caractérisés par le fait que** les anticorps sont des anticorps polyclonaux.

3. Absorbants immunitaires selon la revendication n°2 pour une utilisation conformément à la revendication n°1, **caractérisés par le fait que** les anticorps sont des anticorps aviaires de type IgY.

4. Absorbants immunitaires selon les revendications n°1 à n°3 pour une utilisation conformément à la revendication n°1, **caractérisés par le fait qu'**ils contiennent d'autres anticorps contre des médiateurs d'inflammation selon l'état de régulation de la réaction d'inflammation, ces anticorps étant orientés contre TNF, IL1, IL6, IL8 et/ou IL10.

5. Absorbant immunitaire selon les revendications n°1 et n°4 pour une utilisation conformément à la revendication n°1, **caractérisé par le fait qu'**il contient des anticorps spécifiques à C3a et des anticorps spécifiques à C5a.

6. Absorbant immunitaire selon les revendications n°1 et n°4 pour une utilisation conformément à la revendication n°1, **caractérisé par le fait qu'**il contient des anticorps qui sont orientés contre C3a et/ou C5a ainsi que des anticorps spécifiques à TNF.

7. Absorbant immunitaire selon les revendications n°1 et n°4 pour une utilisation conformément à la revendication n°1, **caractérisé par le fait qu'**il contient des anticorps qui sont orientés contre C3a et/ou C5a ainsi que des anticorps spécifiques à IL1.

8. Absorbant immunitaire selon les revendications n°1 et n°4 pour une utilisation conformément à la revendication n°1, **caractérisé par le fait qu'**il contient des anticorps qui sont orientés contre C3a et/ou C5a ainsi que des anticorps spécifiques à IL6.

9. Absorbant immunitaire selon les revendications n°1 et n°4 pour une utilisation conformément à la revendication n°1, **caractérisé par le fait qu'**il contient des anticorps qui sont orientés contre C3a et/ou C5a ainsi que des anticorps spécifiques à IL8.

10. Absorbant immunitaire selon les revendications n°1 et n°4 pour une utilisation conformément à la revendication n°1, **caractérisé par le fait qu'**il contient des anticorps qui sont orientés contre C3a et/ou C5a ainsi que des anticorps spécifiques à IL10.

11. Absorbant immunitaire selon les revendications n°1 à n°10 pour une utilisation conformément à la revendication n°1, **caractérisé par le fait que** les anticorps liés sont orientés contre au moins une des séquences peptides suivantes des facteurs complémentaires C3a et C5a
C3a:
NH₂-KCCEDGMRQNPMR-COOH
NH₂-RFSCQRRTRFISL-COOH
NH₂-ITELRRQHARAS-COOH
C5a:
NH₂-QADYKDDDDKLPAE-COOH
NH₂-DDKLPAEGLDIENS-COOH

12. Absorbant immunitaire selon les revendications n°1 à n°4 et n°7 pour une utilisation conformément à la revendication n°1, **caractérisé par le fait que** les anticorps liés sont orientés contre au moins une des séquences peptides suivantes des interleukines 1α et 1β
IL1α:
NH2-NCYSENEEDSSSID-COOH
NH₂-GAYKSSKDDAKIT-COOH
NH₂-WETHGTKNYFTS-COOH
IL1β:
NH₂-RISDHHYSKGFRQA-COOH
NH₂-VQGEESNDKIPVA-COOH
NH₂-ESVDPKNYPKKKMEKRF-COOH

13. Absorbant immunitaire selon les revendications n°1 à n°4 et n°8 pour une utilisation conformément à la revendication n°1, **caractérisé par le fait que** les anticorps liés sont orientés contre au moins une des séquences peptides suivantes de l'interleukine 6
IL6:
NH₂-APHRQPLTSSERIDKQI-COOH
NH₂-QNRFESSEEQARA-COOH
NH₂-AITTPDPTTNAS-COOH

14. Absorbant immunitaire selon les revendications n°1 à n°4 et n°10 pour une utilisation conformément à la revendication n°1, **caractérisé par le fait que** les anticorps liés sont orientés contre au moins une des séquences peptides suivantes de l'interleukine 10
IL10:
NH₂-SPGQGTQSENSCT-COOH
NH₂-QMKDQLDNLLLKES-CCOH
NH₂-MPQAENQDPDIKA-COOH
NH₂-LPCENKSKAVEQ-COOH

15. Absorbant immunitaire selon les revendications n°1 à n°4 et n°6 pour une utilisation conformément à la revendication n°1, **caractérisé par le fait que** les anticorps liés sont orientés contre au moins une des séquences peptides suivantes
TNFα:
NH₂-VRSSSRTPSDKPVA-COOH
NH₂-KSPCQRETPEGAEAKPW-COOH

16. Absorbant immunitaire selon les revendications n°1 à n°15 pour une utilisation conformément à la revendication n°1, **caractérisé par le fait que** le matériau support organique ou synthétique se compose de membranes ou de particules de polystyrènes, d'hydrates de carbone, tels que les dérivés de cellulose ou d'agarose, ou d'acrylates.

17. Absorbant immunitaire selon les revendications n°1 à n°16 pour une utilisation conformément à la revendication n°1, **caractérisé par le fait que** les anticorps spécifiques sont liés en covalence aux membranes ou particules.

18. Absorbant immunitaire selon les revendications n°1 à n°16 pour une utilisation conformément à la revendication n°1, **caractérisé par le fait que** les anticorps sont fixés aux matériaux supports par l'intermédiaire d'espaceurs ou de lieurs.

19. Procédé de fabrication d'adsorbants immunitaires conformément aux revendications n°1 à n°18, **caractérisé par le fait que** des anticorps qui sont orientés contre
- C3a et C5a ainsi que, le cas échéant, contre TNF, IL1, IL6, IL8 et/ou IL10, ou
- C5a ainsi que contre TNF, IL1, IL6, IL8 et/ou IL10
sont couplés en covalence ou par absorption aux matériaux supports composés de polymères organiques ou synthétiques.

20. Procédé selon la revendication n°19, **caractérisé par le fait que** les anticorps sont fabriqués par le biais de l'immunisation de petits mammifères, de préférence, tels que les souris, les rats ou les lapins ou d'oiseaux, tels que les poules, avec les antigènes appropriés.

21. Emploi d'adsorbants immunitaires conformément aux revendications n°1 à n°18, faisant partie intégrante d'un dispositif destiné à éliminer des facteurs complémentaires et, le cas échéant, d'autres médiateurs combinés aux liquides organiques spécifiques du patient.

22. Emploi selon la revendication n°21, **caractérisé par le fait que** les adsorbants immunitaires font partie intégrante d'un dispositif destiné à traiter des patients souffrant de septicémie ou sous choc septicémique ainsi que d'autres maladies qui sont accompagnées d'inflammations.
